# EUROPÄISCHE PATENTSCHRIFT

(11) **EP 1 955 051 B1**
(45) Veröffentlichungstag und Bekanntmachung des Hinweises auf die Patenterteilung: **07.01.2015**
(21) Anmeldenummer: 06828570.9
(22) Anmeldetag: 29.11.2006
(51) Int. Cl.: G01N 21/55, G01N 27/12, G01N 33/00, G01N 21/78

(54) **Wasserstoffsensor**
Hydrogen sensor
Détecteur d'hydrogène

(30) Priorität: 29.11.2005 DE 102005057214
(43) Veröffentlichungstag der Anmeldung: 13.08.2008
(73) Patentinhaber: Justus-Liebig-Universität Gießen, 35390 Gießen (DE)
(72) Erfinder: FARANGIS, Baker, 35390 Giessen (DE); MEYER, Bruno, K., 35440 Grossen-Linden (DE); JORDE, Hans-Peter, 35463 Fernwald (DE); STIEBICH, Jennifer, 35447 Reiskirchen (DE)
(74) Vertreter: Patentanwälte Olbricht Buchhold Keulertz
(86) Internationale Anmeldenummer: PCT/DE2006/002109
(87) Internationale Veröffentlichungsnummer: WO 2007/062629

(56) Entgegenhaltungen:
- DE-A1- 10 145 719
- US-A1- 5 591 321
- BODZENTA J ET AL: "Thin palladium film as a sensor of hydrogen gas dissolved in transformer oil" SENSORS AND ACTUATORS B, ELSEVIER SEQUOIA S.A., LAUSANNE, CH, Bd. 87, Nr. 1, 15. November 2002 (2002-11-15), Seiten 82-87, XP004391080 ISSN: 0925-4005
- YOSHIMURA K ET AL: "ROOM-TEMPERATURE HYDROGEN SENSOR BASED ON PD-CAPPED MG2NI THIN FILM" JAPANESE JOURNAL OF APPLIED PHYSICS, JAPAN SOCIETY OF APPLIED PHYSICS, TOKYO, JP, Bd. 43, Nr. 4B, 15. April 2004 (2004-04-15), Seiten L507-L509, XP001222348 ISSN: 0021-4922
- FARANGIS B ET AL: "Structural and electronic properties of magnesium-3D transition metal switchable mirrors" SOLID STATE IONICS, NORTH HOLLAND PUB. COMPANY. AMSTERDAM, NL, Bd. 165, Nr. 1-4, Dezember 2003 (2003-12), Seiten 309-314, XP004476918 ISSN: 0167-2738
- RICHARDSON T J ET AL: "Mixed metal films with switchable optical properties" APPLIED PHYSICS LETTERS, AIP, AMERICAN INSTITUTE OF PHYSICS, MELVILLE, NY, US, Bd. 80, Nr. 8, 25. Februar 2002 (2002-02-25), Seiten 1349-1351, XP012031636 ISSN: 0003-6951

## Beschreibung

### Beschreibung und Stand der Technik

Die vorliegende Erfindung betrifft eine neuartige Klasse von Gas-Sensoren, insbesondere für Wasserstoff oder Wasserstoff enthaltende Fluide (Gase oder Flüssigkeiten), wie z.B. Kohlenwasserstoffe, Schwefelwasserstoffe oder komplexere Gasgemische oder Gaszusammensetzungen enthaltend Wasserstoff oder WasserstoffVerbindungen.

Solche Sensoren finden in vielen Bereichen, z.B. der Industrie und dort z.B. in der chemischen Industrie oder in der Halbleiterfertigung eine Anwendung.

Im Stand der Technik sind bereits verschiedenste Gassensoren auch für die Messung von Wasserstoff enthaltenden Gasen bekannt. So zeigt z.B. die DE 102 61 299 bekannte Gassensoren, welche für den Nachweis brennbarer Gase oxidionenleitende Festelektrolyten (wie z.B. Zirkoniumoxid) nutzen.

Andere Gassensoren nutzen -wie z.B. in der US 5 591 321- Metall-Isolator-Halbleiter-Übergänge (MIS-Sensoren) zum Nachweis von Gasen.

Auch bekannt sind solche Gassensoren, welche die Festkörperelektrolyse als Messprinzip nutzen.

Wieder andere Gassensoren -wie z.B. in DE 101 45 719 gezeigt- nutzen optische Untersuchungsverfahren zum Nachweis von Gasen.

Während letzt genannte den Vorteil besitzen, dass keinerlei Gefährdung dadurch von der Messung ausgeht, dass elektrische Ströme (Funkengefahr) oder hohe Temperaturen (Festkörperelektrolyse bei z.B. 600°C) innerhalb der hochexplosiven Gasumgebung benötigt werden, weist auch die letzt genannte Sensorart den Nachteil auf, dass die Messungen recht aufwendig sind. Es bedarf dazu sehr aufwendiger Apparaturen.

Auch weisen alle vorgenannten Sensor-Arten den Nachteil auf, dass der Nachweis von Gas entweder über anzulegende Spannungen oder Ströme oder aber nur über optische Nachweise erfolgt.

Ein einfacher, im Betrieb und der Produktion kostengünstiger, verlässlicher Sensor, welche für optische oder elektrische "Auslesung" -ohne bauliche Änderung des Sensors als solchem- geeignet ist, ist bislang vollkommen unbekannt.

Die Arbeit von K. Yoshimura, Y. Yamada, M. Okada, M. Tazawa und P. Jin (Room-Temperature Hydrogen Sensor Based on Pd-Capped Mg2Ni Thin Film. Japanese Journal of Applied Physics. Vol. 43, 2004, L507-L509) zeigt einen Wasserstoff-Sensor, der mit einem Palladium versehenen Dünnschichtfilm aus Mg₂Ni ausgestattet. Der Sensor besteht aus einem Glasssubstrat, einer funktionellen Mg₂Ni-Schicht und einer Palladium-Schicht. Die Palladium-Schicht dient dabei als Mittel zur Aktivierung oder Spaltung von Wasserstoff.

### Aufgabe der Erfindung

Aufgabe der Erfindung ist es daher, eine neue Art von Gassensoren anzugeben, welche die vorgenannten Nachteile im Stand der Technik vermeidet und wobei eine besonders hohe "Standzeit" des Sensors erreicht wird.

Weiterhin ist es Aufgabe der vorliegenden Erfindung ein Verfahren zur Messung von wasserstoffhaltigen Gasen anzugeben.

Die erste Aufgabe wird erfindungsgemäß gelöst durch den Gegenstand von Anspruch 1. Die zweite Aufgabe wird gelöst durch den Gegenstand von Anspruch 9.

Bei einem erfindungsgemäßen Gassensor besteht die funktionelle Schicht aus MgAl oder MgAl(₁₋ₓ)Niₓ.

Zur Ausbildung eines eigenständigen Sensors hat man gefunden, dass ein einfacher Sensor folgenden Aufbau aufweisen sollte:
a) Substrat,
b) Funktionelle Schicht oder funktioneller Bereich
c) Mittel zur Aktivierung oder Spaltung von Wasserstoff

In einer besonders bevorzugten Ausführungsform, welche sowohl optisch als auch elektronisch ausgelesen werden kann,
besteht das Substrat aus einem transparenten Material, wie z.B. Glas oder Kunststoff oder Polymeren oder Halbleitern.

Bei dieser Ausführungsform sind auch keine Mittel zur elektronischen Kontaktierung notwendig.

Die funktionelle Schicht oder der funktionelle Bereich besteht in der optischen Ausführungsform oder der elektrischen Ausführungsform aus MgAl oder MgAl₍₁₋ₓ₎Niₓ.

Die Mittel zur Aktivierung oder zur Spaltung oder Dissoziation (im obigen Sinne) von Wasserstoff können -so der Sensor als Schichtsystem ausgeführt ist- in Form einer Deckschicht aus Pd oder Pt oder anderen wasserstoffaktivierenden oder wasserstoffspaltenden oder -dissozierenden Schichten ausgeführt sein, welche oberhalb der funktionellen Schicht angeordnet sind.

Diese Mittel müssen so ausgeführt sein, dass der aktivierte oder gespaltene oder dissozierte Wasserstoff noch in Wechselwirkung mit der aktiven Schicht treten kann, so dass eine Messung des Produktes der Wasserstoffaktivierung oder -spaltung oder -dissoziation erfolgen kann.

Eine besonders bevorzugte Ausführungsform, welche eine besonders hohe "Standzeit" des Sensors aufweist (d.h. der Sensor kann -im Vergleich zu anderen Sensoren- ohne Beeinträchtigung der Messfunktion, besonders lange auch sauerstoffhaltiger oder anderer oxidierender Umgebung ausgesetzt sein) weist neben Magnesium noch Aluminium als Bestandteil der funktionellen Schicht auf. Neben Aluminium sind auch andere Fängermittel für oxidierende Substanzen einsetzbar, wie z.B. Vanadium oder Li oder Na geeignet, sofern diese in der Lage sind Sauerstoff oder Sauerstoffverbindungen oder andere Oxidationsmittel zu binden oder zu absorbieren und damit einer "Vergiftung" (z.B. "Sauerstoffvergiftung) der eigentlich sensitiven Magnesiumteile des Sensors vorzubeugen oder diese Vergiftung zumindest hinaus zu zögern.

Die Untersuchungen in welchen Mg und Al als Bestandteil der funktionellen Schicht oder der funktionellen Bereiche verwendet wurde oder wurden, wiesen als weiteren -gegenüber Sensoren mit Mg und Ni in amorpher, mikrokristalliner oder teilweise polykristalliner Form in der funktionellen Schicht- Vorteil auf, dass die Schaltzeiten bei den Ausführungsformen mit Aluminium deutlich kürzer sind.

In der elektrischen Ausführungsform sind zwischen dem Substrat und der funktionellen Schicht oder dem funktionellen Bereich noch Elektroden oder Leiterbahnen angeordnet, welche die Messung einer elektrischen Spannung oder deren Veränderung über die funktionelle Schicht oder die funktionellen Bereiche hinweg ermöglichen. In der rein elektrischen Ausführungsform kann auch das Substrat aus -für Licht- undurchsichtigem Material bestehen.

In der kombiniert optischen und elektrischen Ausführungsform sollten die Elektroden oder die entsprechend einsetzbaren Leiterbahnen so ausgeführt oder angeordnet sein, dass -für den Fall der optischen Auslesung- das verwendete Licht zumindest auf einen Teil der funktionellen Schicht fallen kann. D.h. die Leiterbahnen oder - flächen oder Elektroden müssen entweder durchsichtig für das betreffende Licht sein, oder dürfen nicht die gesamte Fläche der aktiven Schicht oder aktiven Bereiche abdecken. Falls die wasserstoffaktivierende, -spaltende oder -dissoziierende Schicht für das betreffende Licht transparent ausgeführt ist, können die Leiterbahnen oder - flächen auch undurchsichtig für das betreffende Licht ausgeführt sein. Gleiches gilt dann für das Substrat.

Eine besondere Ausführungsform der rein optischen Ausführungsform weist im Falle von Glas als Substrat oder vergleichbaren Substraten noch eine Nukleations- oder Haftungsschicht für die funktionelle Schicht oder die funktionellen Bereiche auf. Dafür kommen z.B. TiOₓ oder SiOₓ in Frage. Diese Nukleationsschicht kann auch in der rein elektrischen Ausführungsform eingesetzt werden.

Im Folgenden werden weitere Ausführungsbeispiele gezeigt.

Es zeigen die folgenden Figuren:
Fig. 1 die optische Response des erfindungsgemäßen Gassensors in der optischen Ausführungsform nach Zuführung (Öffnung des Ventils) von Wasserstoff
Fig. 2 die optische Response eines Sensors gemäß Fig. 1 bei Zugabe unterschiedlicher Wasserstoff-Konzentrationen.

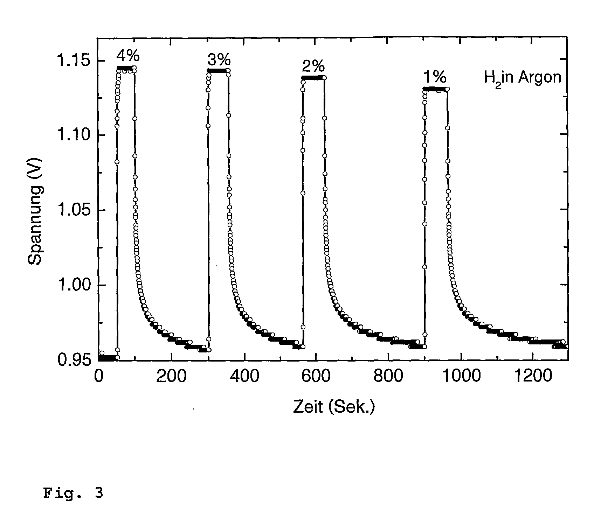

Fig. 3 die elektrische Response, d.h. Änderung der Spannung bei Zugabe unterschiedlicher Wasserstoff-Konzentrationen.

Die vorgenannten funktionellen Schichten, vorzugsweise in rein amorpher, mikrokristalliner und/oder teilweise polykristalliner Ausführung, die z.B. über Sputterverfahren oder andere Dünnschicht-Abscheidungsverfahren, z.B. auf Glassubstraten abgeschieden werden, können in Wechselwirkung mit Wasserstoff (Metallhydride) sowohl elektrochrome als auch gasochrome Eigenschaften haben.

Bei der Beladung mit Wasserstoff ändern sich die elektrischen Eigenschaften (Widerstand) oder aber auch die optischen Eigenschaften (hoch reflektierender, nicht transmittierender in einen transmittierenden, niedrig reflektierenden Zustand). Diese Eigenschaften sind über die Zusammensetzung der eigentlichen funktionalen Schicht in einer Dicke von z.B. zwischen 30-50 nm (Magnesium-Nickel-Legierung) mit einer katalytischen Palladiumdeckschicht, z.B. zwischen 7-10 nm, einstellbar. So ist der transmittierende Zustand praktisch unabhängig von der Wellenlänge. Widerstandsänderungen oder optische Eigenschaftsänderungen und ihr zeitlicher Response auf die Wasserstoffzugabe können über die Zusammensetzung (Mg vs Ni oder Mg vs Al in freier Kombination), Schichtdicke u.a.m. gesteuert werden. Demzufolge können zwei verschiedene Typen von Sensoren realisiert werden.

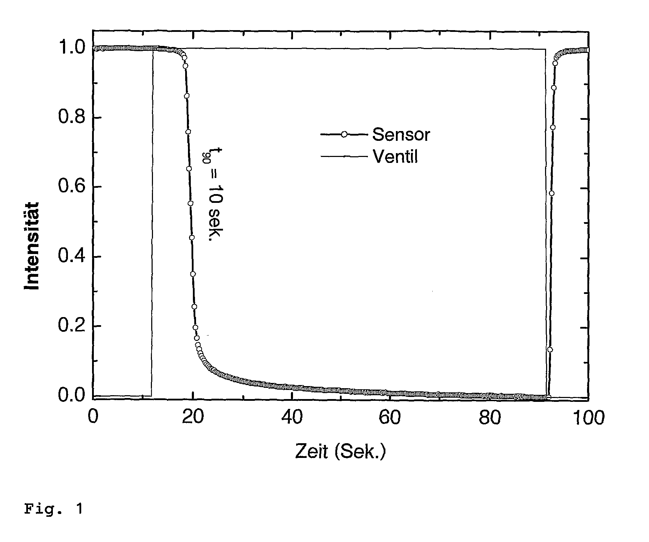

### Zu Fig. 1: Optischer Sensor

Bei dieser Methode wird die Änderung der optischen Eigenschaften, d.h. z.B. die Transmissionsänderung während der Wasserstoffabsorption detektiert. Als Lichtquelle dient in diesem Beispiel eine GaAs-IR-Lumineszenzdiode und als Detektor z.B. ein NPN-Silizium-Fototransistor, der die reflektierte Intensität misst. Die Probengröße lag bei 6 mm x 6 mm.

Fig. 1 zeigt eine Messung mit 4% Wasserstoff in Argon bei Raumtemperatur. Die Signalintensität vor und nach der Wasserstoffabsorption bleibt stabil und die Absorption des Wasserstoffs erfolgt rasch und erreicht innerhalb von 10 Sekunden 90% des Maximalwertes (t₉₀). Vergleichbare Messungen mit vergleichbaren Ergebnissen wurden mit verschiedensten transparenten Substraten, neben dem hier verwendeten Glas durchgeführt und auch mit Wasserstoff in anderen Gasen als dem hier verwendeten Ar durchgeführt.

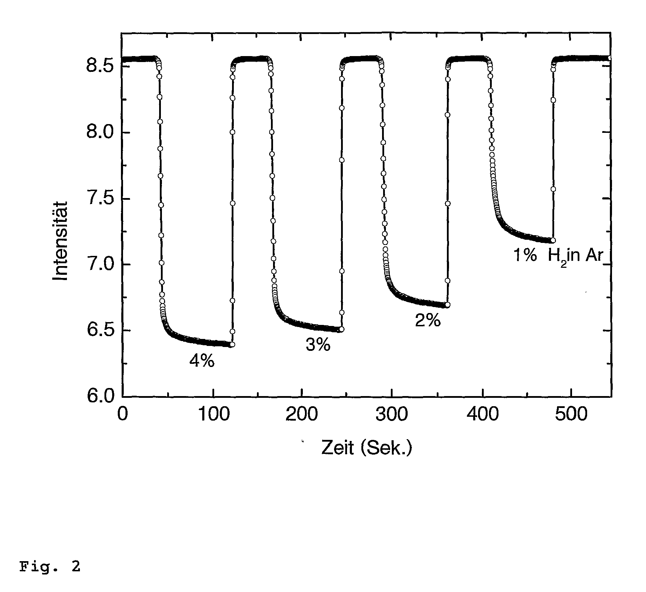

Fig. 2 zeigt die Empfindlichkeit des Sensors auf unterschiedlichen Wasserstoffgehalt von 1-4%, wobei die unter Nachweisgrenze im Bereich von ca. 0.1 % lag. In späteren Versuchen wurde festgestellt, dass die Nachweisgrenze noch unterhalb von 1/100% liegt.

In nicht gesondert dargestellten Versuchen wurden für Messungen analog nach Fig. 2, für einen Mg-Fe-Gassensor t90(sek)-Schaltzeiten von weniger als 5s , 9s, 13s für 1%, 0,5% und 0,1% bei Spannungsdifferenzen von 930, 775 und 600 mV und für einen Mg-Zn Sensor t90-Schaltzeiten von weniger als 10s, 18s, 35s bei Spannungsdifferenzen von 450, 215 und 85 mV bei gemessen.

Zudem betrifft die Erfindung ein neuartiges Verfahren zur Messung von Wasserstoff oder wasserstoffhaltigen Verbindungen, wie z.B. Kohlenwasserstoffen oder Schwefelwasserstoffen.

Dabei wird das zu messende Gas, enthaltend Wasserstoff oder wasserstoffhaltige Verbindungen, einem erfindungsgemäßen Gassensor zugeführt, wobei die Detektion des Wasserstoff-Gehaltes oder des Gehaltes an wasserstoffhaltigen Verbindungen unter Aufnahme oder Abgabe der vorgenannten Gase auf oder von MgAl oder MgAl₍₁₋ₓ)Niₓ erfolgt.

Besonders bevorzugt verläuft das neuartige Messverfahren wie oben, jedoch abgeändert darum, dass gleichzeitig oder periodisch oder wiederholt bei der Aufnahme oder/und Abgabe auf den vorgenannten Substanzen ein Fängermittel zur Vermeidung der Vergiftung des Mg, wie z.B. Al und/oder Vanadium und/oder Li und/oder Na im zu messenden oder bestimmenden Gasfluss oder in der zu messenden Umgebung bereitgestellt wird. Dadurch wird die Standzeit des Sensors deutlich erhöht.

Die Herstellung der neuartigen Sensor-Klasse erfolgt wie folgt:
Optische Ausführungsform: Die funktionelle Schicht kann auf einer Substratschicht aus gängigem transparentem Material wie z.B. Glas oder Plexiglas oder nicht transparentem Material aufgebracht werden.

Zur Herstellung wird das Substrat mit einem der bekannten Dünnschicht-Depositionsverfahren, z.B. mit der Sputtertechnik, der chemischen Abscheidung aus Gasphasen (CVD, MOCVD), dem Solgelverfahren, durch Aufdampfen, durch Pyrolyse mit der funktionellen Schicht oder mit den funktionellen Bereichen beschichtet.

Eine Abwandlung des Verfahrens zur Herstellung der optischen Ausführungsform und damit ein vorteilhaftes Ausführungsbeispiel zu dem hier genannten Verfahren ergibt sich, wenn zunächst auf das Substrat eine Nukleationsschicht aufgebracht wird, auf welche die Funktionsschicht aufgebracht wird. Als Nukleationsschicht eignen sich besonders solche, die eine ähnliche Struktur und Gitterkonstante, inbesondere bei der Depositionstemperatur der Funktionsschicht, aufweisen.

Zur Herstellung der elektrischen Ausführungsform kann nach bekannten Verfahren wie z.B. thermischem Verdampfen, Sputtertechnik, einer chemischen Abscheidung aus Gasphasen (CVD, MOCVD), dem Solgel-Verfahren, durch Aufdampfen, durch Pyrolyse oder durch nasschemisches Abscheiden oder durch Elektrolyse zunächst eine elektrisch leitende Schicht auf das Substrat gebracht werden, oder eine leitende Schicht ebenfalls durch die bekannten oder oben genannten Dünnschicht-Depositionsverfahren auf das Substrat aufgebracht werden. Bei der rein elektrischen Ausführungsform ist keine Haftungs- oder Nukleationsschicht notwendig, daher kann auf die elektrische leitende Schicht oder die Elektroden oder elektrischen Leiter dann direkt mittels der bekannten oder oben genannten Dünnschicht-Depositionsverfahren die funktionelle Schicht aufgebracht werden.

Anschließend ist auf diese -bei allen Ausführungsformen- (d.h. rein optisch, oder rein elektrisch oder kombiniert, optisch oder elektrisch auszulesender Sensor) die Wasserstoff-Aktivierungs- oder Wasserstoff-Spaltungs oder -Dissoziations-Schicht durch oben genannten oder bekannten Dünnschicht-Depositionsverfahren aufzubringen.

### Bezugszeichenliste

- 1: Substrat
- 2: Funktionelle Schicht oder Bereiche
- 3: Aktivierungs- oder Spaltungsschicht
- 4: Elektroden oder elektrische Leiterbahnen
- 5: Nukleations- oder Haftungsschicht

## Patentansprüche

1. Gassensor zur Bestimmung von Wasserstoff oder Wasserstoffhaltigen Verbindungen in Gasen oder Fluiden, umfassend ein Substrat, eine funktionelle Schicht und ein Mittel zur Aktivierung oder Spaltung von Wasserstoff, **dadurch gekennzeichnet, dass** die funktionelle Schicht aus MgAl oder MgAl₍₁₋ₓ)Niₓ besteht.

2. Gassensor entsprechend Anspruch 1, **dadurch gekennzeichnet, dass** der Sensor ein Schichtsystem ist.

3. Gassensor entsprechend einem der Ansprüche 1 oder 2, **dadurch gekennzeichnet, dass** das Mittel zur Aktivierung oder Spaltung von Wasserstoff eine Deckschicht aus Pd, Pt oder einem anderen wasserstoffaktivierende, -spaltenden oder - dissoziierenden Material ist.

4. Gassensor entsprechend einem der Ansprüche 1 bis 3, **dadurch gekennzeichnet, dass** der Sensor elektrisch und/oder optisch auslesbar ist, wobei die elektrische Auslesung über die Widerstandsänderung der funktionellen Schicht erfolgt und wobei die optische Auslesung über die Änderung des Reflexionsgrades der funktionellen Schicht erfolgt.

5. Gassensor entsprechend Anspruch 4, **dadurch gekennzeichnet, dass** zwischen dem Substrat und der funktionellen Schicht Elektroden oder Leiterbahnen angeordnet sind.

6. Gassensor entsprechend Anspruch 5, **dadurch gekennzeichnet, dass** die Elektroden oder Leiterbahnen durchsichtig für das verwendete Licht sind.

7. Gassensor entsprechend einem der Ansprüche 1 bis 6, **dadurch gekennzeichnet, dass** das Substrat durchsichtig für das verwendete Licht ist.

8. Gassensor entsprechend einem der Ansprüche 1 bis 7, **dadurch gekennzeichnet, dass** zwischen dem Substrat und der funktionellen Schicht eine Nukleationsschicht angeordnet ist.

9. Verfahren zur Messung der Konzentration oder zum Nachweis von Wasserstoff oder wasserstoffhaltigen Verbindungen, **dadurch gekennzeichnet, dass** das zu messende Gas einem Gassensor nach einem der Ansprüche 1 bis 8 zugeführt wird und die Messung oder der Nachweis unter Aufnahme oder Abgabe der Gase auf oder von MgAl oder MgAl₍₁₋ₓ₎Niₓ erfolgt.

10. Verwendung einer der Sensoren nach den Ansprüchen 1 bis 8 in der Halbleiter- oder chemischen Industrie als Gas- oder Rauchmelder, zur Überwachung von Verbrennungsprozessen, in Brennstoffzellen oder Brennstofftanks oder bei der Elektrolyse oder Zerlegung von Wasser.

## Claims

1. Gas sensor for detecting hydrogen or hydrogen-containing compounds in gases or fluids, comprising a substrate, a functional layer and a means for activating or cleaving hydrogen, **characterised in that** the functional layer consists of MgAl or MgAl₍₁₋ₓ₎Niₓ.

2. Gas sensor according to claim 1, **characterised in that** the sensor is a layer system.

3. Gas sensor according to one of claims 1 or 2, **characterised in that** the means for activating or cleaving hydrogen is a covering layer of Pd, Pt or another hydrogen-activating, -cleaving or -dissociating material.

4. Gas sensor according to one of claims 1 to 3, **characterised in that** the sensor is electrically and/or optically readable, the electrical reading being obtained by means of the change in resistance of the functional layer and the optical reading being obtained by means of the change in the reflectance of the functional layer.

5. Gas sensor according to claim 4, **characterised in that** electrodes or strip conductors are arranged between the substrate and the functional layer.

6. Gas sensor according to claim 5, **characterised in that** the electrodes or strip conductors are translucent to the light used.

7. Gas sensor according to one of claims 1 to 6, **characterised in that** the substrate is translucent to the light used.

8. Gas sensor according to one of claims 1 to 7, **characterised in that** a nucleation layer is arranged between the substrate and the functional layer.

9. Method for measuring the concentration of or for detecting hydrogen or hydrogen-containing compounds, **characterised in that** the gas to be measured is fed into a gas sensor according to one of claims 1 to 8, and the measurement or detection is carried out as the gases are absorbed on or given off by MgAl or MgAl₍₁₋ₓ₎Niₓ.

10. Use of one of the sensors according to one of claims 1 to 8 in the semiconductor or chemical industry as a gas or smoke detector, for monitoring combustion processes, in fuel cells or fuel tanks or in the electrolysis or decomposition of water.

## Revendications

1. Détecteur de gaz pour la détermination d'hydrogène ou de composés contenant de l'hydrogène dans des gaz ou des fluides, comprenant un substrat, une couche fonctionnelle et un moyen d'activation ou de clivage de l'hydrogène, **caractérisé en ce que** la couche fonctionnelle consiste en MgAl ou MgAl(₁₋ₓ)Niₓ.

2. Détecteur de gaz selon la revendication 1, **caractérisé en ce que** le détecteur est un système à couche.

3. Détecteur de gaz selon l'une des revendications 1 ou 2, **caractérisé en ce que** l'agent d'activation ou de clivage de l'hydrogène est une couche de revêtement de Pd, Pt ou d'un autre matériau activant, clivant ou dissociant l'hydrogène.

4. Détecteur de gaz selon l'une des revendications 1 à 3, **caractérisé en ce que** le détecteur peut être consulté électriquement et/ou optiquement, la consultation électrique s'effectuant par modification de la résistance de la couche fonctionnelle et la consultation optique s'effectuant par la modification du degré de réflexion de la couche fonctionnelle.

5. Détecteur de gaz selon la revendication 4, **caractérisé en ce qu'**entre le substrat et la couche fonctionnelle sont disposées des électrodes ou des pistes conductrices.

6. Détecteur de gaz selon la revendication 5, **caractérisé en ce que** les électrodes ou les pistes conductrices sont transparentes pour la lumière utilisée.

7. Détecteur de gaz selon l'une des revendications 1 à 6, **caractérisé en ce que** le substrat est transparent pour la lumière utilisée.

8. Détecteur de gaz selon l'une des revendications 1 à 7, **caractérisé en ce qu'**entre le substrat et la couche fonctionnelle est disposée une couche de nucléation.

9. Procédé de mesure de la concentration ou de la détection d'hydrogène ou de composés contenant de l'hydrogène, **caractérisé en ce que** le gaz à mesurer est acheminé à un détecteur de gaz selon l'une des revendications 1 à 8, et la mesure ou la détection s'effectue par absorption ou restitution des gaz sur ou de MgAl ou MgAl(₁₋ₓ)Niₓ.

10. Utilisation de l'un des détecteurs selon les revendications 1 à 8, dans l'industrie des semiconducteurs ou l'industrie chimique en tant que détecteur de gaz ou détecteur de fumée, pour la surveillance de processus de combustion, dans des piles à combustibles ou des réservoirs de combustibles ou dans l'électrolyse ou la décomposition de l'eau.
